# EUROPEAN PATENT APPLICATION

(11) **EP 1 974 603 A2**
(43) Date of publication of application: **01.10.2008**
(21) Application number: 08153321.8
(22) Date of filing: 26.03.2008
(51) Int. Cl.: A01K 11/00, A44C 5/00, A61B 5/117, G04B 37/14, G06K 19/00, G09F 3/00, H04B 1/38

(54) **Identification band with flattened ID portion for facilitated and improved read-out of printed information**

(30) Priority: 26.03.2007 US 908104 P; 25.03.2008 US 55088
(71) Applicant: PRECISION DYNAMICS CORPORATION, San Fernando, CA 91340 (US)
(72) Inventor: Bekker, Alexander, Sherman Oaks, CA 91423 (US)
(74) Representative: Richter, Werdermann, Gerbaulet & Hofmann

(57) **Abstract**

The identification band (20) includes a strap (28) adjacent to a first end (24) and an information-bearing portion integral therewith and adjacent to a second end (26). Attachments means allow for the first end to be looped around the person or object to be identified and attached to the strap portion (28) so that the information-bearing portion (30) is not part of the loop (34), but extends away from the loop (34) around the person or object identified. The band includes a slot (52) through which the first end is passed prior to being attached to the strap. By extending away from the loop (34), the information-bearing portion is able to maintain a flattened and enlarged surface area from which barcode or human-readable information (32) can be more easily scanned and/or read.

## Description

### BACKGROUND OF THE PRESENT INVENTION

Hospital bracelets have been in use for many years, with on demand and barcode printing bands increasingly demanded. One drawback associated with newborn and infant ID bands is that the band that fits their limb is narrow, because wide bands are uncomfortable to the infant's skin. Limited human readable data can be printed on a bracelet that is small and narrow and the curvature of a band wrapped around an infant's small limb may be too extreme to take a proper barcode reading.

### DISCLOSURE (SUMMARY) OF THE PRESENT INVENTION: PROBLEM, SOLUTION, ADVANTAGES

Accordingly, there is a need for a newborn and infant ID band that provides a broader information-bearing surface and avoids the curvature to the information-bearing surface resulting when it is applied to an infant's small limb. The present invention fulfills these needs and provides other related advantages.

The identification band of the present invention is a single-use bracelet that includes an adhesive, snap, or other closure which permits the bracelet to be secured to a person of object to be identified. One novel feature of the infant ID band is an information-bearing portion integral with the band's construction but not part of the bracelet formed around the infant's limb. A tail end of the band is inserted through a slot near the information-bearing portion and adhesively (or by a snap closure) attached to the top surface of the band. This construction allows for the information-bearing portion to remain straight extending away from the infant's limb and the band to be adjusted to the size of the infant's limb. By not being a part of the bracelet formed around the infant's limb, the information-bearing surface is able to maintain a flattened and enlarged surface area from which barcode or human-readable information can be more easily scanned and/or read.

The present invention is directed to an identification band comprising an elongated wristband having opposite first and second ends, a strap portion adjacent to the first end and an information bearing portion adjacent to the second end. The band also includes an adhesive or snap closure mechanism for attaching the first end to the strap portion to form a loop for encircling a person or object to be identified. The band may also include a slot therethrough such that the first end is passed through the slot before being attached to the strap portion.

Where an adhesive closure is used, the band may also include tamper cuts associated therewith. The tamper cuts are designed to reveal attempts at removing the band once it is attached.

The identification band may be presented in a continuous tape comprising a plurality of identification bands. On such a tape, a locator opening or locator mark may be included to help a printer align the tape with the print head. The identification band in tape form is comprised of a thermo-sensitive top ply laminated to a polymer bottom ply, usable with thermal printers.

The identification band may also be presented in a sheet including a plurality of identification bands and/or a plurality of labels or cards. In sheet form, the elongated wristband is comprised of a laser printable top ply laminated to a polymer bottom ply, usable with laser printers.

Other features and advantages of the present invention will become apparent from the following more detailed description, taken in connection with the accompanying drawings, which illustrate, by way of example, the principles of the present invention.

### BRIEF DESCRIPTION OF THE DRAWINGS

The accompanying drawings illustrate the invention. In such drawings:

FIG.1 illustrates a preferred embodiment of an identification band embodying the present invention;

FIG. 2 illustrates the inventive identification band of FIG. 1 forming a loop using an adhesive closure;

FIG.3 illustrates an alternate embodiment of an identification band embodying the present invention;

FIG. 4 illustrates the inventive identification band of FIG. 3 forming a loop using a slot and a snap closure;

FIG. 5 illustrates another alternate embodiment of an identification band embodying the present invention;

FIG. 6 illustrates the inventive identification band of FIG. 5 forming a loop using a slot and an adhesive closure;

FIG. 7 illustrates a thermal printer tape of identification bands embodying the present invention;

FIG. 8 illustrates a laser printer sheet of identification bands embodying the present invention including associated labels;

FIG. 9 illustrates a laser printer sheet of identification bands embodying the present invention including multiple bands;

FIG. 10 illustrates a roll of a thermal printer tape of identification bands embodying the present invention loaded in a thermal printer;

FIG. 11 sheets of identification bands embodying the present invention loaded in a laser printer;

FIG. 12 illustrates the layered construction of a tape of identification bands embodying the present invention;

FIG. 13 illustrates a cross-section of a tape form of identification bands taken along line 13-13 of FIG. 7;

FIG. 14 illustrates the layered construction of a sheet of identification bands embodying the present invention;

FIG. 15 illustrates a partial cross-section of a sheet form of identification bands taken along line 15-15 of FIG. 8;

FIG. 16 illustrates the front of a tape of identification bands embodying the present invention;

FIG. 17 illustrates the back of a tape of identification bands embodying the present invention;

FIG. 18 illustrates the front of a sheet of identification bands embodying the present invention; and

FIG. 19 illustrates the back of a sheet of identification bands embodying the present invention.

### DETAILED DESCRIPTION OF THE PREFERRED EMBODIMENTS

As shown in the exemplary drawings, for purposes of illustration, the present invention is concerned with a novel identification band generally referred to by reference numeral 20. Different embodiments of the inventive identification band 20 will be described below. Through the description of each embodiment, the same reference numerals will be used to refer to similar components, including the identification band 20.

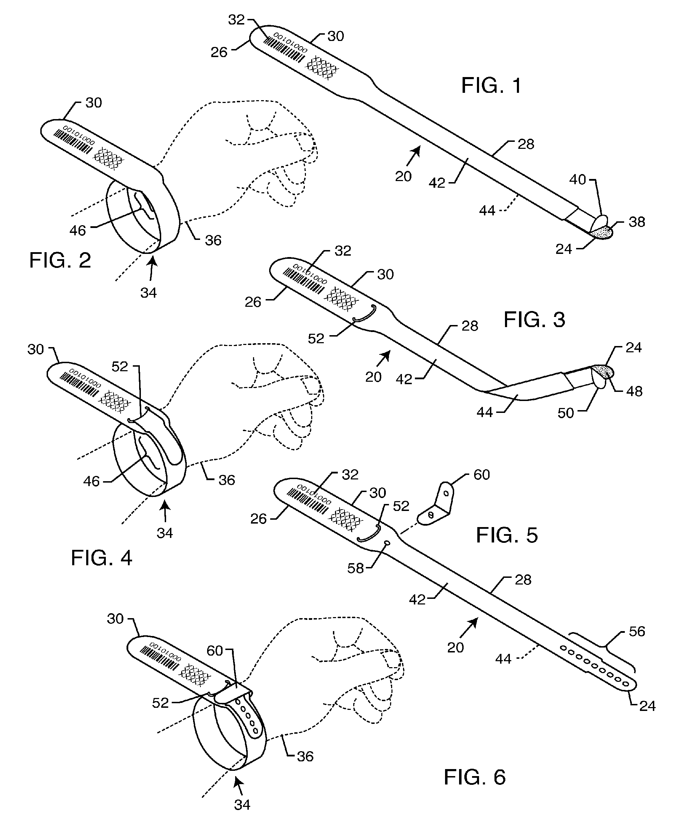

As depicted in FIGS 1, 3 and 5, every embodiment of the inventive identification band 20 is an elongated wristband having a first end 24 and a second end 26. The identification band 20 includes a strap portion 28 adjacent to the first end 24 and an information bearing portion 30 for receiving printed information or identification data 32 integral with the identification band 20 and adjacent to the second end 26. One of the novel features of the identification band 20 is the manner in which it forms a loop 34 for securing around the wrist 36 of a person to be identified. In the inventive identification band 20, the loop 34 is formed such that the information bearing portion 30, while being integral with the identification band 20, is not part of the loop 34, but extends away from the wrist 36. In this way, the information bearing portion 30 is able to maintain a flattened and enlarged surface area from which the printed information 32 (barcode, words and/or numbers) is more easily scanned and/or read.

The identification bands 20 are formed from a flexible, non-stretchable, machine-imprintable material. As illustrated in FIGS 1 thru 6, information bearing portion 30 is rounded and rectangular and the elongated strap portion 28 is narrower than the information bearing portion 30. The information bearing portion 30 and strap portion 28 are arranged along a common centerline, but may also be asymmetrical. The strap portion 28 may be approximately one-half the width of the information bearing portion 30. Other relative widths of the two portions 28, 30 may be used such that the information bearing portion 30 is sufficiently wide to bear human-readable and/or machine-readable information and the strap portion 28 is sufficiently narrow to be comfortable when applied to the wrist 36 of a person, i.e, an infant.

While the inventive identification band 20 is described in terms of being secured to the wrist 36 of a person, one having ordinary skill in the art will realize that the band 20 may be secured in a similar manner to another portion of a person or even an object to be identified while employing the same principles.

In a first embodiment, depicted in FIGS 1 and 2, the first end 24 of the identification band 20 includes an adhesive portion 38 having a liner tab 40 on an upper surface 42 of the strap portion 28. The information bearing portion 30 is oriented such that printed information 32 is received on the upper surface 42. In order to secure this embodiment of the identification band 20 into a loop 34, one must remove the liner tab 40 from the adhesive portion 38 and then loop the strap portion 28 around a wrist 36 and secure the adhesive portion 38 to a lower surface 44 of the identification band 20 at an attachment point 46 on the strap portion 28. This attachment point 46 can be any point along the strap potion 28 so as to create a loop 34 of sufficient size to accommodate the wrist 36 of the person being identified, as depicted in FIG. 2.

Key to the inventive identification band 20 is that the attachment point 46 be on the strap portion 28 and not on the information bearing portion 30. By placing the attachment point 46 on the strap portion 28, the information bearing portion 30 is left to extend away from the wrist 36 rather than be part of the loop 34 and subject to the curvature of the loop 34. One skilled in the art will realize that by not making the information bearing portion 30 part of the loop 34 and thus eliminating curvature thereof, the printed information 32 displayed thereon will be easier to read, both by humans and machines. In addition, the avoidance of curvature of the information bearing portion 30 reduces the risk of damaging the printed information 32 from repeated bending and flexing. Such will also reduce the risk of damaging any electronic components contained therein, i.e., radio frequency identification chips, etc.

In a second embodiment, depicted in FIGS 3 and 4, the first end 24 of the identification band 20 includes an adhesive portion 48 having a liner tab 50 on the lower surface 44 thereof and the information bearing portion 30 is oriented such that the printed information 32 is received on the upper surface 42 thereof. In this embodiment, the identification band 20 also includes a slot 52 which passes through from the lower surface 44 to the upper surface 42. The slot 52 is positioned on the identification band 20 adjacent to the junction between the strap portion 28 and the information bearing portion 30. In forming a loop 34, one must pass the first end 24 of the identification band 20 through the slot 52, remove the liner tab 50, and adhere the adhesive portion 48 to the strap portion 28 on the upper surface 42 of the identification band 20.

Since the slot 52 must be wide enough to receive the first end 24 of the identification band 20, i.e., the strap portion 28, it must be placed on the information bearing portion 30 adjacent to the strap portion. In addition, the slot 52 must be positioned so that it does not encroach on the printed information 32 or cause the information bearing portion 30 to become part of the loop and create curvature therein.

In either the first or second embodiments, the first end 24 of the identification band 20 may include tamper cuts 54 overlying the adhesive portions 38, 48. The tamper cuts 52 are configured such that any attempt to open the identification band 20 after being adhered into a loop will destroy the integrity of the identification band 20, thus revealing the attempted opening.

In a third embodiment, depicted in FIGS 5 and 6, the identification band 20 includes a series of openings 56 at the first end 24 and a single opening 58 adjacent the junction between the information bearing portion 30 and the strap portion 28. In forming a loop 34, the first end 24 is looped around and brought adjacent to the lower surface 44 of the other end of the strap portion 28. One of the series of openings 56 is brought into alignment with the single opening 58 such that the loop formed thereby is appropriately sized for the wrist 36. A snap closure mechanism 60 is placed through the aligned openings 56, 58 and then closed. The snap closure mechanism 60 retains the identification band 20 in a closed loop configuration around the wrist 36 of the person to be identified.

Alternatively, the wristband may include a slot 52 as described above. The first end 24 is passed through the slot 52 and brought adjacent to the upper surface 42 of the other end of the strap portion 28. One of the series of openings 56 is again brought into alignment with the single opening 58 and secured by a snap closure mechanism 60. This configuration including the slot 52 functions in a similar manner to the configuration without the slot 52. The use of the slot 52 facilitates applying the snap closure mechanism 60 and creates a slightly more secure loop 34.

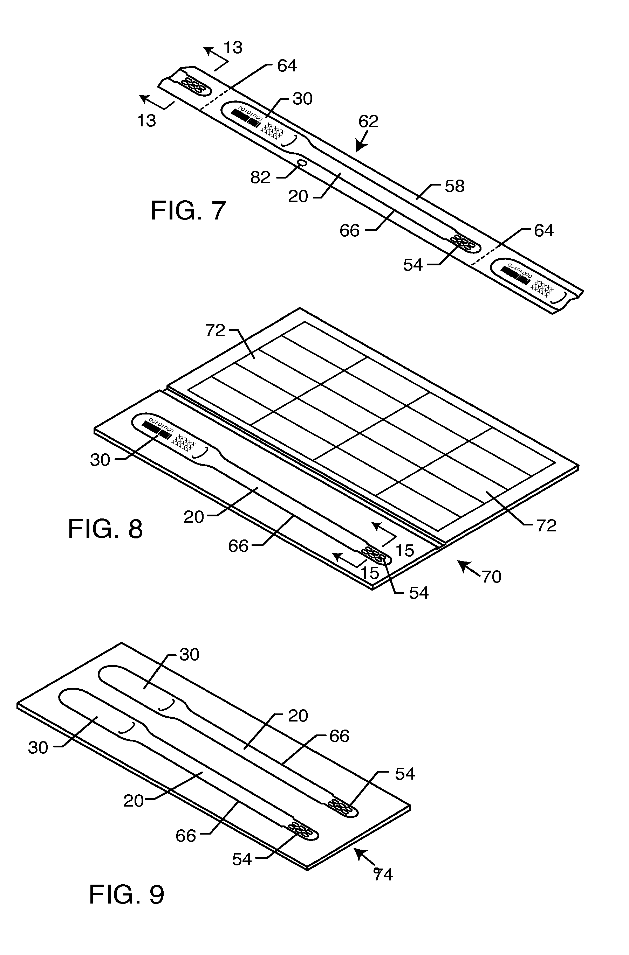

The identification band 20 may be presented in several formats. As shown in FIG. 7, multiple identification bands 20 may be presented in a continuous tape 62 wherein each band is separated from an adjacent band by perforations 64. The tape 62 has parallel side edges and is therefore of uniform width throughout its full length. The sheets 70, 74 may include one or more bands 20 in conjunction with one or more cards or labels 72. In this embodiment, each identification band 20 is separated from the tape 62 by a line of weakness 66 which follows the perimeter of the identification band 20. It is preferred that the lines of weakness 66 be scored or kiss-cut so as to leave a smooth edge following a tearing operation. Excess tape or waste material 68 may be discarded after the identification band 20 is removed.

As shown in FIG. 8, the identification bands 20 may also be presented in a sheet form 70 that includes an identification band 20 in combination with multiple labels and/or cards 72. An alternate sheet form 74, as shown in FIG. 9, may include multiple identification bands 20. As with the tape form 62, the identification bands 20 are surrounded by lines of weakness 66 for easy separation from the sheets 70, 74. The labels and/or cards 72 are also surrounded by lines of weakness 66 for easy separation from the sheets 70, 74. The detailed construction of the tape form 62 and sheet forms 70, 74 will be described more fully below.

Sets of multiple identification bands 20 may be created either from a sequence of the tape form 62 or a single sheet form 70, 74. The sets may include mother/infant, mother/father/infant or other similarly related sets of persons or objects. In addition, sets may include multiple maternity identification sets consisting of multiple parents and/or multiple infants. In each multiple identification set, the identification bands 20 would each bear matching printed information 32 and electronically-readable data joined together.

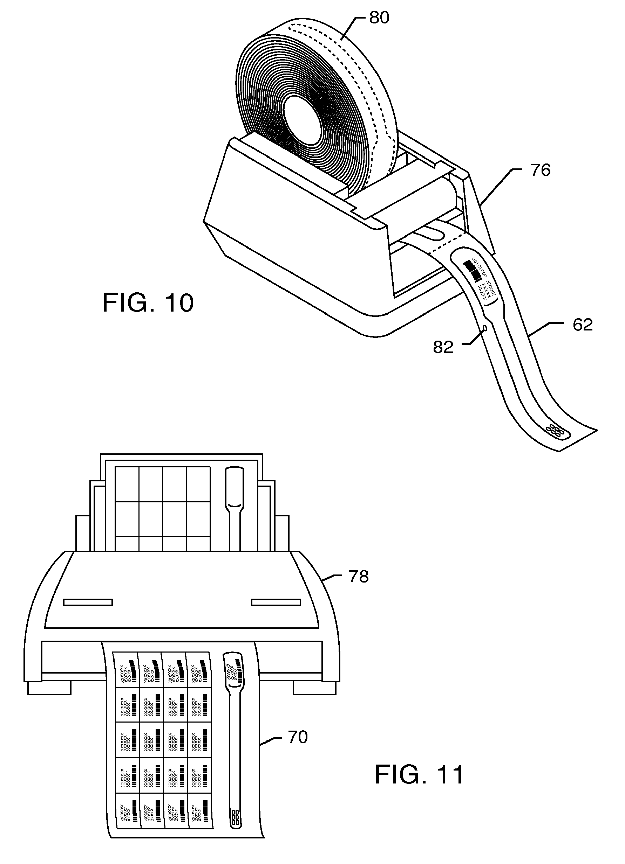

The identification band 20 requires the use of an appropriate printer to print barcode and/or human-readable printed information 32 on the band 20 at the time of issue. The identification bands 20 may be configured to receive printed information 32 either by thermal printer 76, laser printer 78 or similar means. As shown in FIG. 10, when a thermal printer 76 is used the identification bands 20 are preferably supplied in a roll or coil 80 of the tape form 62. When a laser printer 78 is used, the identification bands 20 are preferably supplied in sheet form 70, 74, as shown in FIG. 11. Once printed and issued, data may be captured and processed visually or with available barcode readers.

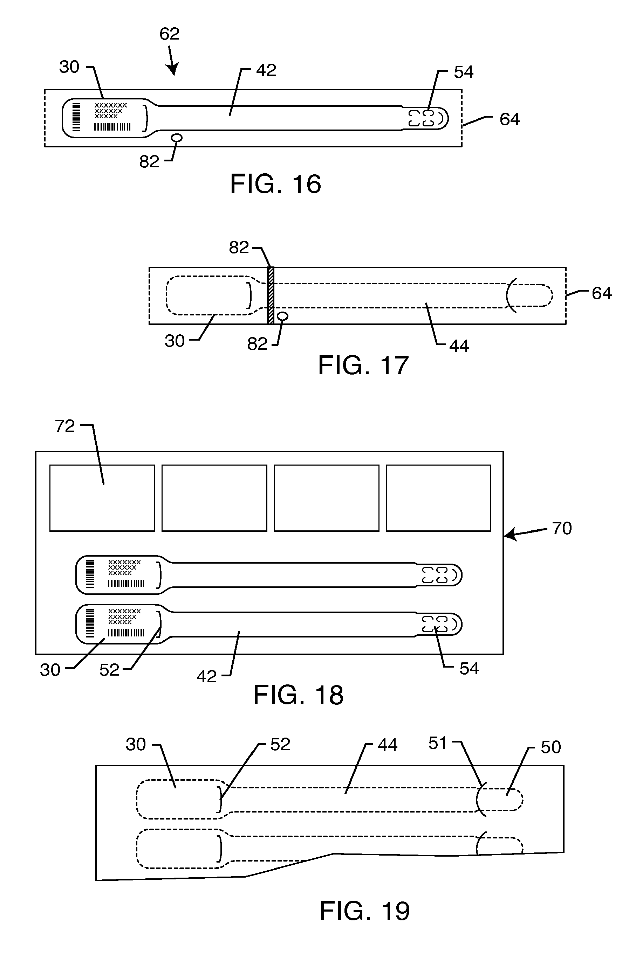

For the thermal printer tape form 62, as shown in FIGS 16 and 17, the back side of the tape adjacent each identification band 20 is provided with a locator opening and/or mark 82 for detection by an optical sensor in the thermal printer 76. This locator opening and/or mark 82 assists in controlling advancement of the tape 62 through the printer 76. The printer 76 may be programmed to automatically control the intermittent advancement of the tape 62 and positioning of the tape 62 when imprinting a band 20 by using this locator opening and/or mark 82. The locator openings 82 are preferably confined to the waste sections 68, outside the limits of the bands 20 so as not to weaken or otherwise affect the integrity of the band 20. Alternatively, a locator mark 82 may be positioned anywhere on the lower surface 44 of the tape 62, because a mark 82 will not weaken or take away from the integrity of the band 20.

FIGS 18 and 19 illustrate front and back views of a sheet 70, 74 of identification bands 20. In FIG. 19, the liner tab 50 is shown with a release cut 51 adjacent thereto. This release cut 51 is designed to allow the liner tab 50 to be removed without tearing or disturbing the adjacent portions of the strap portion 28.

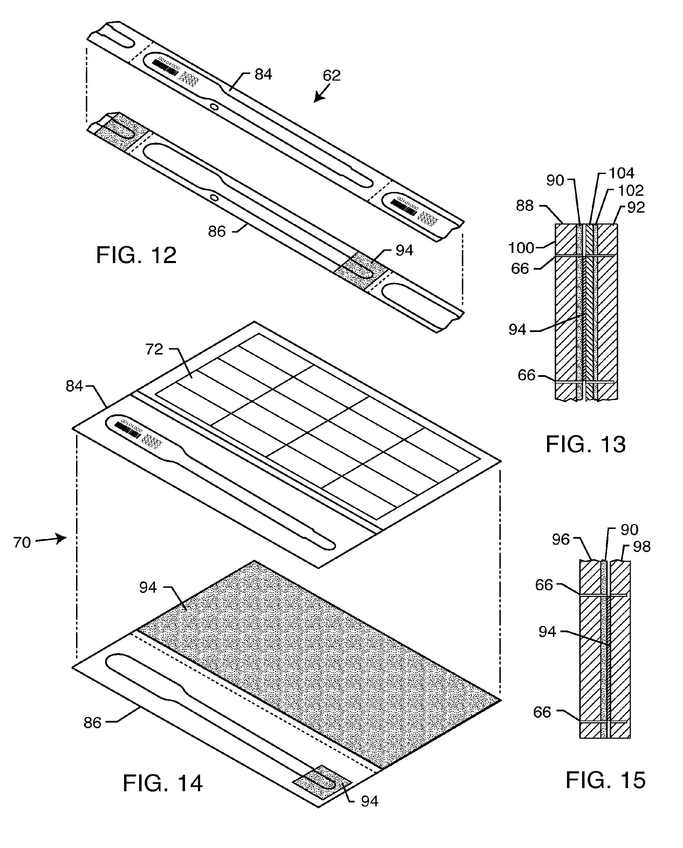

As shown in FIGS 12 thru 15, the identification band 20 is composed of a top ply 84 and a bottom ply 86 laminated together. In the case of a thermal printable tape form 62, the top ply 34 is preferably made from a direct thermo-sensitive stock 88 backed with pressure-sensitive adhesive 90. The top ply 84 preferably includes an overprint varnish 100 on top of the thermo-sensitive stock 88 with the pressure sensitive adhesive 90 thereunder. The bottom ply 86 may be manufactured from a bi-laminate (i.e. made from low density polyethylene and polyester) or single layer film 92. The bottom ply 86 preferably consists of a bottom polyethylene layer 92 with a UV adhesive 102, a top polyester coating 104 and a pattern release coating 94 on one surface directly under the first end 24 of the identification band 20. The top and bottom plies 84, 86 are bonded together using the pressure-sensitive adhesive 90.

In the case of a laser-printable sheet form 70, 74, the top ply 84 is preferably made from a laser-printable face film 96 with a pressure sensitive adhesive 90 on one side. The bottom ply 86 is manufactured from a single layer film (i.e., polyester or similar material to withstand the heat of the laser printer) 98. Again the bottom ply 86 includes a pattern release coating 94 on one surface directly under the first end 24 of the identification band 20. As with the rolled form 62, the top and bottom plies 84, 86 are bonded together using the pressure-sensitive adhesive 90.

The inventive identification band 20 may be used for patient identification in hospitals or similar health care settings. It is specially designed for identification of infant patients, hence the need to prevent curvature of the information bearing portion 30. The identification bands 20 may have the added ability to link their printed information 32, i.e., barcodes, to the electronic tracking of treatments, medications, procedures and other pertinent health information. This tracking assumes the existence of an underlying software system for doing so. General information may be printed on the outside surface of the information bearing portion 30 at the time of issue. Since the barcode variation of the identification band 20 does not require human-readable visibility conditions, information may be accessed with fewer disturbances to the infant patient.

Although various embodiments of the present invention have been described in detail for purposes of illustration, various modifications may be made without departing from the scope and spirit of the invention.

## Claims

1. An identification band, comprising:
an elongated wristband having opposite first and second ends, a strap portion adjacent to the first end and an information bearing portion adjacent to the second end; and
means for attaching the first end to the strap portion to form a loop for encircling a person or object to be identified.

2. The identification band of claim 1, further comprising a slot through the wristband such that the first end is passed through the slot before being attached to the strap portion.

3. The identification band of claim 1, wherein the means for attaching comprises an adhesive or snap closure mechanism.

4. The identification band of claim 1, further comprising tamper cuts associated with the means for attaching.

5. The identification band of claim 1, wherein the identification band is presented in a continuous tape comprising a plurality of identification bands.

6. The identification band of claim 5, further including a locator opening or a locator mark on the tape.

7. The identification band of claim 5, wherein the elongated wristband is comprised of a thermo-sensitive top ply laminated to a polymer bottom ply.

8. The identification band of claim 1, wherein the identification band is presented in a sheet including a plurality of labels or cards.

9. The identification band of claim 8, wherein the elongated wristband is comprised of a laser printable top ply laminated to a polymer bottom ply.

10. The identification band of claim 1, wherein the identification band is presented in a sheet including a plurality of identification bands.

11. An identification band, comprising:
an elongated wristband having opposite first and second ends, a strap portion adjacent to the first end and an information bearing portion adjacent to the second end;
a slot through the wristband such that the first end is passed through the slot to form a loop for encircling a person or object to be identified; and
adhesive means for attaching the first end to the strap portion.

12. The identification band of claim 11, further comprising tamper cuts associated with the adhesive means.

13. The identification band of claim 1, wherein the identification band is presented in a continuous tape comprising a plurality of identification bands.

14. The identification band of claim 13, further including a locator opening or a locator mark on the tape.

15. The identification band of claim 13, wherein the elongated wristband is comprised of a thermo-sensitive top ply laminated to a polymer bottom ply.

16. The identification band of claim 11, wherein the identification band is presented in a sheet including a plurality of labels or cards.

17. The identification band of claim 16, wherein the elongated wristband is comprised of a laser printable top ply laminated to a polymer bottom ply.

18. The identification band of claim 11, wherein the identification band is presented in a sheet including a plurality of identification bands.

19. An identification band, comprising:
an elongated wristband having opposite first and second ends, a strap portion adjacent to the first end and an information bearing portion adjacent to the second end;
a slot through the wristband such that the first end is passed through the slot to form a loop for encircling a person or object to be identified; and
snap closure means for attaching the first end to the strap portion.

20. The identification band of claim 19, wherein the identification band is presented in a continuous tape comprising a plurality of identification bands.

21. The identification band of claim 20, further including a locator opening or a locator mark on the tape.

22. The identification band of claim 20, wherein the elongated wristband is comprised of a thermo-sensitive top ply laminated to a polymer bottom ply.

23. The identification band of claim 19, wherein the identification band is presented in a sheet including a plurality of labels or cards.

24. The identification band of claim 23, wherein the elongated wristband is comprised of a laser printable top ply laminated to a polymer bottom ply.

25. The identification band of claim 19, wherein the identification band is presented in a sheet including a plurality of identification bands.
